# EUROPEAN PATENT APPLICATION

(11) **EP 2 277 437 A1**
(43) Date of publication of application: **26.01.2011**
(21) Application number: 09166079.5
(22) Date of filing: 22.07.2009
(51) Int. Cl.: A61B 5/00, A61B 5/08

(54) **Method and system for adaptive breath pacing**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention relates to a method for adaptive breath pacing comprising the steps of determining a breathing pattern of a user in a first step, synchronizing an artificial breathing pattern to the determined breathing pattern in a second step, feedback the artificial breathing pattern to the user in a third step, modulating the synchronized artificial breathing pattern in a fourth step and feedback the modified breathing pattern to the user in a fifth step.

## Description

### FIELD OF THE INVENTION

The present invention relates to the field of adaptive breath pacing for inducing a subject to fall asleep or to relax.

### BACKGROUND OF THE INVENTION

Breathing is one of the few bodily functions which, within limits, can be controlled both consciously and unconsciously. It is well known, that correct breathing contributes to health and feelings of well-being, whereby the correct breathing could be influenced by feedback the breathing pattern to the breathing person.

For example, the PCT patent application WO 2007 / 105 127 A1 proposes a system for inducing a subject to fall to sleep, comprising a light pattern generator for generating a time varying light pattern in view of the subject and a breathing rate measuring unit for measuring a breathing frequency of the subject. In addition, the system comprises a control unit connected to the breathing rate measuring unit and the light pattern generator, for controlling the light pattern generator, such that the generated light pattern has a pattern frequency substantially between the measured breathing frequency and a pre-selected desired frequency.

Moreover, the PCT patent application WO 2005 / 089 856 Al discloses a methods and devices for identifying a user's Respiratory Sinus Arrhythmia (RSA) waves during respiration and providing the user with near real-time Respiratory Sinus Arrhythmia (RSA) wave information. This information can be used in biofeedback settings to assist users in reducing levels of stress by achieving rhythmic breathing patterns.

### SUMMARY OF THE INVENTION

It is an object of the present invention to provide a method for adaptive breath pacing which is more effective compared to the prior art and which is highly adaptable to arbitrary breathing patterns and influences the breathing of the user in a very comfortable and suggestive manner without forcing the user's breathing into a fixed and predetermined pattern.

The above mentioned object is accomplished by a method for adaptive breath pacing comprising the steps of determining a breathing pattern of a user in a first step, synchronizing an artificial breathing pattern to the determined breathing pattern in a second step, feedback the artificial breathing pattern to the user in a third step, modulating the synchronized artificial breathing pattern in a fourth step and feedback the modified breathing pattern to the user in a fifth step.

According to the present invention, it is thereby advantageously possible that initially the artificial breathing pattern is synchronized to the detected breathing pattern and only afterwards the artificial breathing pattern is modulated for slowing down the user's breathing frequency. In other words, the manipulation of the user's breathing characteristics starts not until the actual breathing pattern of the user is substantially imitated by the synchronized artificial breathing pattern for at least a certain time period. Consequently, in the third step the user's breathing pattern becomes more regular before the user's breathing pattern is slowing down in the fifth step. As a result, it is significantly easier for the user to engage oneself with the artificial breathing pattern and the effect of inducing the user to fall asleep or to relax is much more effective. Subsequently, the pacing is done without the subject really noticing or having to force into a fixed or predetermined breathing pattern. In fact, there is a slow transition from observing the user's breathing behavior to actively manipulating the user's breathing behavior. The feedback of the artificial breathing pattern comprises preferably visual, tactile and/or audible signals provided to the user. The determined breathing pattern of the user is preferably derived from the Respiratory Sinus Arrhythmia (RSA) wave of the user and is much preferred determined by the aid of a heart rate detector, an Electrocardiograph (ECG), an Electroencephalograph (EEG), a Photoplethysmograph (PPG), a force sensor, a pressure sensor, a strain gauge or the like.

In a preferred embodiment of the present invention a first phase of the breathing pattern is determined in the first step and a second phase of the artificial breathing pattern is synchronized to the first phase in the second step. Beneficially, almost a real-time synchronization between the artificial breathing pattern and the determined breathing pattern is provided, because a whole measurement of the first frequency of the determined breathing pattern is not necessary.

Preferably, the third step is performed for a certain time period and/or until the first phase and the second phase are substantially synchronous to each other over a certain time period and/or the first, the second and the third step are repeated several times, for a certain time period and/or until the first phase and the second phase are substantially synchronous over a certain time period. Consequently, a successful synchronization between the breathing pattern and the artificial breathing pattern is assured. Furthermore, the user is capable of slowly getting accustomed to the feedback by the artificial breathing pattern before the breathing pattern of the user is modulated in the fifth step.

Preferably, the synchronized artificial breathing pattern is modulated by reducing the frequency of the synchronized artificial breathing pattern compared to the primal frequency of the determined breathing pattern in the fourth step. The user's breathing characteristics gets influenced by the artificial breathing pattern in such a manner that also the frequency of the breathing pattern of the users slows down and follows the frequency of the modulated artificial breathing pattern. Particularly, the frequency is lowered smoothly or in steps to a certain target frequency in such a manner that the user begins to settle down, to relax, to reduce stress and/or to fall asleep. It shall be understood that in the meantime the modulated artificial breathing pattern is provided to the user. Preferably, the user is able to choose a certain target frequency via a user interface and/or the target frequency is derived automatically from personal information of the user, like gender, age, weight, height or the like.

In another preferred embodiment of the present invention a phase shift between the first phase of the breathing pattern and the second phase of the modified artificial breathing pattern is determined in a sixth step and wherein the fourth, the fifth and the sixth steps are repeated sequentially or in parallel in such a manner, that in the fourth step the modification of the artificial breathing pattern is performed in dependency of the phase shift determined in the sixth step. If the reduction of the frequency of the artificial breathing pattern will be too fast, a phase shift or respectively a rising phase shift between the first phase of the actual breathing pattern and the second phase of the modulated artificial breathing could arise which lowers the effectiveness of the breath pacing procedure. In order to avoid this, the phase shift between the actual breathing pattern of the user and the modulated breathing pattern is supervised all along, during the artificial breathing pattern gets modulated.

Preferably, the artificial breathing pattern is modulated in the fourth step in such a manner, that the phase shift between the first and the second phase which is determined in the sixth step remains substantially constant and/or below a certain threshold. Advantageously, the user's breathing behavior remains in time with the modulated artificial breathing patterns and consequently the manipulation of the user's breathing pattern based on the feedback of the modulated artificial breathing pattern to the user is comparatively effective.

Another object of the present invention is a system for adaptive breath pacing comprising a sensor unit for determining a breathing pattern of a user, a control unit for providing an artificial breathing pattern, a synchronizer unit for synchronizing the artificial breathing pattern to the determined breathing pattern, an modulator unit for modulating the artificial breathing pattern and an output unit for feedback the artificial breathing pattern to the user. Advantageously, the system is capable of synchronizing the artificial breathing pattern to the determined breathing pattern before the artificial breathing pattern is modulated for manipulating the user's breathing behavior.

Preferably, the system comprises a timer unit for initiating the modulation of the synchronized artificial breathing pattern as soon as the synchronized artificial breathing pattern has been provided to the user for a certain time period. The output unit comprises a loudspeaker, a light source, a vibrating device, for instance, wherein the sensor unit preferably comprises a heart rate sensor, an Electrocardiograph, an Electroencephalograph, a Photoplethysmograph, a force sensor, a pressure sensor, a strain gauge, or the like.

These and other characteristics, features and advantages of the present invention will become apparent from the following detailed description, taken in conjunction with the accompanying drawing, which illustrates, by way of example, the principles of the invention. The description is given for the sake of example only, without limiting the scope of the invention. The reference figures quoted below refer to the attached drawing.

### BRIEF DESCRIPTION OF THE DRAWINGS

- Figure 1: shows a system for adaptive breath pacing according to an exemplary embodiment of the present invention;
- Figure 2: shows a breathing pattern of a user and an artificial breathing pattern provided by a method according to an exemplary embodiment of the present invention;

### DETAILED DESCRIPTION OF EMBODIMENTS

The present invention will be described with respect to particular embodiments and with reference to a certain drawing but the invention is not limited thereto but only by the claims. The drawing described is only schematic and is non-limiting. In the drawing, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated. Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein. It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

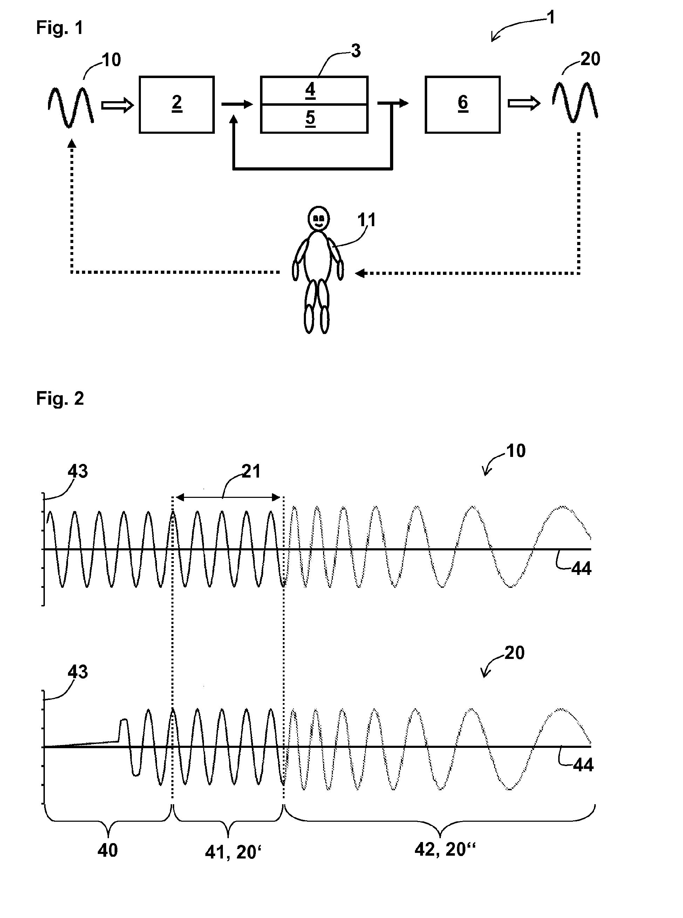

Figure 1 shows a system 1 for adaptive breath pacing according to an exemplary embodiment of the present invention comprising a sensor unit 2, a control unit 3 and an output unit 6. The sensor unit 2 comprises e.g. an electrocardiograph (ECG) for determining a breathing pattern 10 of a user 11, wherein the Respiratory Sinus Arrhythmia (RSA) waveform of the breathing pattern 10 is derived. The control unit 3 provides a corresponding artificial breathing pattern 20 which is provided to the user 11 via the output unit 6. The output unit 6 comprises e.g. a loudspeaker which plays back a faked breathing sound with the parameters of the artificial breathing pattern 20 as a biofeedback to the user 11. The artificial breathing pattern 20 is furthermore feed back to a synchronizer 4 which is a part of the control unit 3. The synchronizer 4 determines a phase shift between a first phase of the determined breathing pattern 10 and a second phase of the artificial breathing pattern 20. Subsequently, the synchronizer 4 synchronizes the artificial breathing pattern 20 to the determined breathing pattern 10, so that the user 11 gets a feedback with the synchronized artificial breathing pattern 20, 20' which is in time with his personal breathing behavior. The feedback subtly influences the user 11 to engage his breathing behavior to the synchronized artificial breathing pattern 20 and consequently to breathe more regular. After a certain time period 21, a modulator unit 5 which is a part of the control unit 3 modulates the synchronized artificial breathing pattern 20, 20' by slowly and evenly reducing the frequency of the artificial breathing pattern 20, 20' to a certain target frequency. The modulated artificial breathing pattern 20, 20" slowly lowering its frequency is permanently provided to the user 11. As a result, the user's breathing behavior is manipulated in such a manner, that the user 11 similarly reduces the frequency of his breathing pattern 10 in the same way.

Consequently, the user 11 relaxes and falling asleep of the user 11 is at least assisted. During the modulation of the frequency of the modulated artificial breathing pattern 20, 20" the sensor unit 2 and the control unit 3 preferably supervises the phase shift between the breathing pattern 10 and the modulated breathing pattern 20, 20" to assure that the breathing pattern 10 and the modulated breathing pattern 20, 20" remains in phase.

Figure 2 shows a breathing pattern 10 of a user 11 and an artificial breathing pattern 20 provided by a method according to an exemplary embodiment of the present invention. Each of the determined breathing pattern 10 and the artificial breathing pattern 20 comprises a harmonic Respiratory Sinus Arrhythmia (RSA) wave, whose amplitude 43 is plotted against time 44. In Figure 2 three different time periods 40, 41, 42 are illustrated. In the first time period 40 the initial breathing pattern 10 of the user 11 is determined and analyzed. Furthermore, the artificial breathing pattern 20 has just been provided, wherein the artificial breathing pattern 20 is synchronized to the determined breathing pattern 10 in such a manner, that the artificial breathing pattern 20 is in phase with the determined breathing pattern 10 at least at the end of the first time period 40. Subsequently, the synchronized artificial breathing pattern 20, 20' is provided to the user 11 during the whole second time period 41. As a result, the user is capable of engaging himself with the artificial breathing pattern 20. In the third time period 42, the frequency of the artificial breathing pattern 20 is slowly reduced with time in such a manner that the modulated artificial breathing pattern 20, 20" remains in phase with the determined breathing pattern 10. Consequently, lowering the frequency of the modulated artificial breathing pattern 20, 20" manipulates the user's breathing behavior, so that the user 11 similarly reduces the frequency of his breathing pattern 10. A suchlike manipulated breathing behavior of the user 11 causes the user 11 to relax and to fall asleep more easily. Preferably, the phase shift between the breathing pattern 10 and the modulated breathing pattern 20" is supervised in the third time period 42 to assure that the breathing pattern 10 and the modulated breathing pattern 20, 20" remains in phase.

## Claims

1. Method for adaptive breath pacing comprising the steps of determining a breathing pattern (10) of a user (11) in a first step, synchronizing an artificial breathing pattern (20) to the determined breathing pattern (10) in a second step, feedback the artificial breathing pattern (10) to the user (11) in a third step, modulating the synchronized artificial breathing pattern (20, 20') in a fourth step and feedback the modified breathing pattern (20, 20") to the user (11) in a fifth step.

2. A method according to claim 1, wherein in the first step a first phase of the breathing pattern (10) is determined and wherein in the second step a second phase of the artificial breathing pattern (20) is synchronized to the first phase.

3. Method according to one of the preceding claims, wherein the third step is performed for a certain time period (21) and/or until the first phase and the second phase are substantially synchronous to each other over a certain time period (21).

4. Method according to one of the preceding claims, wherein the first, the second and the third step are repeated several times, for a certain time period (21) and/or until the first phase and the second phase are substantially synchronous over a certain time period (21).

5. Method according to one of the preceding claims, wherein in the fourth step the synchronized artificial breathing pattern (20, 20') is modulated by reducing the frequency of the synchronized artificial breathing pattern (20, 20') compared to the primal frequency of the determined breathing pattern (10).

6. Method according to claim 5, wherein the fourth and the fifth steps are repeated sequentially or in parallel in such a manner, that the frequency of the modulated artificial breathing pattern (20, 20") is reduced smoothly or in steps to a certain target frequency.

7. Method according to one of the preceding claims, wherein in a sixth step a phase shift between the first phase of the breathing pattern (10) and the second phase of the modified artificial breathing pattern (20, 20") is determined and wherein the fourth, the fifth and the sixth steps are repeated sequentially or in parallel in such a manner, that in the fourth step the modification of the artificial breathing pattern (20) is performed in dependency of the phase shift determined in the sixth step.

8. Method according to claim 7, wherein in the fourth step the modulated artificial breathing pattern (20) is modulated in such a manner, that the phase shift between the first and the second phase determined in the sixth step remains substantially constant and/or below a certain threshold.

9. Method according to one of the preceding claims, wherein the feedback of the artificial breathing pattern (20) comprises visual, tactile and/or audible signals provided to the user (11).

10. Method according to one of the preceding claims, wherein in the first step the Respiratory Sinus Arrhythmia (RSA) wave of the user (11) is determined.

11. Method according to one of the preceding claims, wherein in the first step the breathing pattern (10) is determined by the aid of a heart rate detector, an Electrocardiograph (ECG), an Electroencephalograph (EEG), a Photoplethysmograph (PPG), a force sensor, a pressure sensor, a strain gauge, or the like.

12. System (1) for adaptive breath pacing by performing a method according to one of preceding claims comprising a sensor unit (2) for determining a breathing pattern (10) of a user (11), a control unit (3) for providing an artificial breathing pattern (20), a synchronizer unit (4) for synchronizing the artificial breathing pattern (20) to the determined breathing pattern (10), an modulator unit (5) for modulating the artificial breathing pattern (20) and an output unit (6) for feedback the artificial breathing pattern (20) to the user (11).

13. System according to claim 12, wherein the system comprises a timer unit for initiating the modulation of the synchronized artificial breathing pattern (20') as soon as the synchronized artificial breathing pattern (20') has been provided to the user (11) for a certain time period (21).

14. System according to one of the claims 12 or 13, wherein the output unit (6) comprises a loudspeaker, a light source, a vibrating device or the like.

15. System according one of the claims 12 to 14, wherein the sensor unit (2) comprises a heart rate sensor, an Electrocardiograph (ECG), an Electroencephalograph (EEG), a Photoplethysmograph (PPG), a force sensor, a pressure sensor, a strain gauge, or the like.
